# EUROPEAN PATENT APPLICATION

(11) **EP 3 714 823 A1**
(43) Date of publication of application: **30.09.2020**
(21) Application number: 17929538.1
(22) Date of filing: 27.10.2017
(51) Int. Cl.: A61B 18/12, A61B 18/14

(54) **LOW TEMPERATURE PLASMA SNARE KNIFE SURGICAL DEVICE, SYSTEM AND METHOD**

(71) Applicant: Neowing Medical Co., Ltd., Shanghai 201210 (CN)
(72) Inventor: YAN, Hang, Shanghai 201210 (CN); ZHENG, Zhongwei, Shanghai 201210 (CN)
(74) Representative: Zaboliene, Reda
(86) International application number: PCT/CN2017/107942
(87) International publication number: WO 2019/080078

(57) **Abstract**

The present invention discloses a low temperature polypectomy snare surgical device, system and method. The device includes: a liquid input unit, configured to input liquid to a target object in response to a liquid input signal to form a thin layer of a conductive medium between an emission electrode and a loop electrode; a bipolar electrode socket connector, connected with a high frequency generator through a high frequency cable to receive a first input voltage generated by the high frequency generator; the emission electrode, configured to receive the first input voltage generated by the high frequency generator via the bipolar electrode socket connector and apply a first voltage between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma; and the loop electrode, introduced through the same catheter as the emission electrode and forming a conductive loop in the target object.

## Description

### Field of the Invention

The present invention relates to the field of radio frequency technology, and more particularly to a low temperature polypectomy snare surgical device, system and method.

### Background of the Invention

With the continuous development of digestive endoscopy technology, the endoscopic treatment of diseases of digestive tract is becoming more and more popular. The electric snare is used in conjunction with an endoscope to remove polyps or other redundant tissue in the digestive tract by use of high frequency current. It can eliminate the risks of traditional surgical treatment and has the characteristics of little trauma, good curative effect, high requirements on surgical skills, etc.

A high-frequency snare-cutting electric knife is an electrical surgical instrument that replaces a mechanical scalpel for tissue snare cutting. The working principle of the high-frequency snare-cutting electric knife is to heat a tissue when a high-frequency and high-voltage current generated by an effective electrode tip contacts a body so as to separate and coagulate the tissue, thereby achieving purposes of snare cutting and hemostasis. The peak voltage of an electrocoagulation mode of the high-frequency snare-cutting electric knife is greater than that of an electric cutting mode, the high-frequency current generates heat in the tissue while flowing by the high-impedance tissue, resulting in vaporization or coagulation of the tissue, so that a good hemostasis effect is generated, but more obvious thermal damage is generated at the same time. The temperature of the high-frequency snare-cutting electric knife can be instantly stabilized up to more than 150°C. The heating effect of the high-frequency snare-cutting electric knife can snare cut the tissue, but it is not caused by a heating electrode or a knife head. The high-frequency current with high current density is gathered to directly destroy the tissue in contact with the effective electrode tip. When the temperature of the tissue or cells in contact with or adjacent to the effective electrode rises to a protein denaturation temperature in the cells, snare cutting and coagulation effects are generated.

The working temperature of a common high-frequency snare-cutting electric knife is usually 100-150°C, this working temperature is still a high temperature relative to human tissues, after the tissue cells are affected by this temperature, protein denaturation of the tissues is caused by the snare cutting. In particular, the common high-frequency snare-cutting electric knife will cause thermal damage to the tissues after continuously working for a certain period of time. The degenerative necrosis of the tissue cells is a gradual development process. The common high-frequency snare-cutting electric knife will result in reactions such as swelling in a surgical area, postoperative pain, and the like.

In an actual environment, the application of the high-frequency snare-cutting electric knife is prone to complications in the snare-cutting surgery of such as polyps or prominent tumor sites in the digestive tract in hospital, because the temperature will cause damage to the tissues. The high-frequency snare cutting electric knife has two electrodes, one electrode is attached to the body of the patient, the other electrode is placed at the position of the snare, and an electrical path is arranged on the handle. The high-frequency emission temperature as high as 400-500°C will cause accidental damage to the surrounding good tissues, such that the occurrence probability of the bleeding problem is high, and the pathological tissues are damaged easily. In this case, a doctor cannot perform pathological analysis, and it is troublesome to perform effective analysis on slicing. The invention with an application number CN201180055284.9 discloses a bipolar snare, including: a long tubular electrically insulated sheath, a pair of long flexible conductive wires disposed in the sheath, and an electrically insulated connector disposed at a distal end of the wire. The electrically insulated connector mechanically connects the distal ends of the pair of wires to form a ferrule projecting from the distal end of the sheath. The wire is provided with an electrical insulator covering all except one of the selected portions of each of the wires. The bipolar snare device further includes a guide member positioned in the sheath and forming a compartment for each of the electrically conductive wires, the guide member can rotate in the sheath. The guide member also moves backwards from the distal end of the sheath to the extent that the entire ferrule including at least a portion of the connector can be received within the sheath. However, the bipolar snare cannot avoid the problem of accidental injury caused by excessive temperature.

### Summary of the Invention

According to one aspect of the present invention, a low temperature polypectomy snare surgical device is provided, including:
a liquid input unit, configured to input liquid to a target object in response to a liquid input signal to form a thin layer of a conductive medium between an emission electrode and a loop electrode;
a bipolar electrode socket connector, connected with a high frequency generator through a high frequency cable to receive a first input voltage generated by the high frequency generator;
the emission electrode, configured to receive the first input voltage generated by the high frequency generator via the bipolar electrode socket connector and apply a first voltage between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma; and
the loop electrode, introduced through the same catheter as the emission electrode and forming a conductive loop in the target object.

The bipolar electrode socket connector receives a second input voltage generated by the high frequency generator and transmits the second input voltage to the emission electrode, and applies a second voltage between the emission electrode and the loop electrode, so that the target object is maintained at a second temperature so as to cause ablation and coagulation of the target object.

The emission electrode of the low temperature polypectomy snare surgical device is connected to the bipolar electrode socket connector through an emission electrode conducting wire, the emission electrode conducting wire is coated with an insulating layer, and the insulating layer is configured for insulating and thermal insulation.

The low temperature polypectomy snare surgical device further includes a liquid injection cavity configured to input the liquid to the liquid input unit based on a liquid input instruction, wherein the liquid input unit measures a current residual amount of the liquid in real time and sends the current residual amount to a control unit, and the control unit determines whether to generate the liquid input instruction based on the current residual amount and sends the liquid input instruction to the liquid input unit after determining to generate the liquid input instruction.

The liquid input unit performs liquid input in one of a titration mode and a continuous liquid supply mode, and the liquid injection cavity is an annular cavity located at the outside of the loop electrode.

The snare head of the emission electrode away from the emission electrode of the low temperature polypectomy snare surgical device is elliptical, hexagonal or half-moon-shaped.

The liquid input port of the liquid input unit is located between the emission electrode and the loop electrode.

The low temperature polypectomy snare surgical device further includes a pull rod for enabling an operator to provide a supporting force by holding the pull rod, and the low temperature polypectomy snare surgical device further includes an outer tube for providing an outer layer coating function.

The emission electrode of the low temperature polypectomy snare surgical device is telescopically arranged through a slide block.

The range of the first voltage is 100 Vrms to 300 Vrms, and the range of the second voltage is 60 Vrms to 80 Vrms.

Preferably, the range of the first voltage is 100 Vrms to 300 Vrms, and the range of the second voltage is 60 Vrms to 80 Vrms.

According to one aspect of the invention, a low temperature polypectomy snare surgical system is provided, including:
an input unit, configured to receive a control instruction input by a user and send the control instruction to a control unit;
the control unit, configured to parse the control instruction, generate a first mode instruction when the control instruction indicates a first mode, calculate the output power in the first mode according to the current impedance and the control instruction, and send the first mode instruction and a first voltage indication associated with the output power in the first mode to an interface unit;
the interface unit, configured to receive the first mode instruction and the first voltage indication from the control unit, forward the first mode instruction and the first voltage indication to a plasma unit, receive the current impedance of a target contact end from a plasma unit and send the current impedance to the control unit; and
the plasma unit for entering the first mode in response to the reception of the first mode instruction and the first voltage indication from the interface unit, in which mode circuit activation is performed between an emission electrode and a loop electrode at the target contact end of the plasma unit via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma;
wherein the emission electrode, the plasma layer, the loop electrode and the target contact end form a loop.

The control unit further parses the control instruction, generates a second mode instruction when the control instruction indicates a second mode, calculates the output power in the second mode according to the current impedance and the control instruction, and sends the second mode instruction and the second voltage indication associated with the output power in the second mode to the interface unit.

The interface unit receives the second mode instruction and the second voltage indication from the control unit and forwards the second mode instruction and the second voltage indication to the plasma unit.

The plasma unit enters the second mode in response to the reception of the second mode instruction and the second voltage indication from the interface unit, applies a second voltage to maintain the target contact end of the plasma unit at a second temperature so as to perform ablation and coagulation of the target object.

The low temperature polypectomy snare surgical system further includes an alarm unit, configured to perform alarm via voice prompt, character prompt and/or indicator lamp display when an alarm signal is received;
wherein the plasma unit sends an alarm signal to the control unit after detecting an operation fault, and the control unit sends the alarm signal to the alarm unit.

The input unit is a pedal type input device, wherein the user generates the control instruction by operating the pedal type input device, and the control instruction is a two-tuples <mode, power>.

The low temperature polypectomy snare surgical system further includes a dropping liquid input unit for inputting the conductive medium to the plasma unit based on a conductive medium input instruction of the control unit, wherein the plasma unit measures a current residual amount of the conductive medium in real time and sends the current residual amount to the control unit, and the control unit determines whether to generate the conductive medium input instruction based on the current residual amount and sends the conductive medium input instruction to the dropping liquid input unit after determining to generate the conductive medium input instruction.

The low temperature polypectomy snare surgical system further includes a display unit for displaying an operation state of the low temperature polypectomy snare surgical system in real time. The range of the first voltage is 100 Vrms to 300 Vrms, and the range of the second voltage is 60 Vrms to 80 Vrms.

The range of the first temperature is 35°C-40°C, and the range of the second temperature is 40°C-70°C, and
in the first mode, a thermal penetration distance is less than or equal to 150 microns, and in the second mode, the thermal penetration distance is less than or equal to 200 microns.

According to another aspect of the present invention, a low temperature polypectomy snare surgical method is provided, including:
receiving a control instruction input by a user;
parsing the control instruction, generating a first mode instruction when the control instruction indicates a first mode, calculating the output power in the first mode according to the current impedance and the control instruction, and determining a first voltage indication associated with the output power in the first mode;
forwarding the first mode instruction and the first voltage indication to a plasma device, and receiving the current impedance of a target contact end from the plasma device;
causing the plasma device to enter the first mode in response to the reception of the first mode instruction and the first voltage indication, in which mode circuit activation is performed between an emission electrode and a loop electrode at the target contact end of the plasma unit via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma;
wherein the emission electrode, the plasma layer, the loop electrode and the target contact end form a loop.

The low temperature polypectomy snare surgical method further includes: parsing the control instruction, generating a second mode instruction when the control instruction indicates a second mode, calculating the output power in the second mode according to the current impedance and the control instruction, and determining a second voltage indication associated with the output power in the second mode;
forwarding the second mode instruction and the second voltage indication to the plasma device;
causing the plasma device to enter the second mode in response to the reception of the second mode instruction and the second voltage indication, in which mode a second voltage is applied to maintain the target contact end of the plasma unit at a second temperature so as to perform ablation and coagulation of the target object.

The low temperature polypectomy snare surgical method further includes: performing alarm via voice prompt, character prompt and/or indicator lamp display when an alarm signal is received; and generating the alarm signal after detecting an operation fault.

The user generates the control instruction by operating a pedal type input device, and the control instruction is a two-tuples <mode, power>.

The low temperature polypectomy snare surgical method further includes: inputting the conductive medium to the plasma device based on a conductive medium input instruction, wherein the plasma unit measures a current residual amount of the conductive medium in real time and determines whether to generate the conductive medium input instruction based on the current residual amount. The low temperature polypectomy snare surgical method further includes: displaying an operation state of the plasma device in real time.

The range of the first voltage is 100 Vrms to 300 Vrms, and the range of the second voltage is 60 Vrms to 80 Vrms.

The range of the first temperature is 35°C-40°C, and the range of the second temperature is 40°C-70°C, and in the first mode, a thermal penetration distance is less than or equal to 150 microns, and in the second mode, the thermal penetration distance is less than or equal to 200 microns.

The working temperature of the low temperature polypectomy snare surgical system and method according to the present application is only 40-70°C, thereby solving the problem of accidental injury to surrounding good tissues and the bleeding problem, and reducing complications. In addition, the low temperature polypectomy snare surgical system and method of the present application adopt a bipolar mode on the structure to form a loop directly on the catheter.

### Brief Description of the Drawings

Exemplary embodiments of the present invention can be more completely understood with reference to the following drawings:
Fig.1 is a schematic diagram of main parts of a plasma therapeutic apparatus according to a preferred embodiment of the invention;
Fig.2 is a structure diagram of a plasma therapeutic apparatus according to a preferred embodiment of the invention;
Fig.3 is a structure diagram of a low temperature polypectomy snare surgical system according to a preferred embodiment of the invention;
Fig.4 is a flowchart of a low temperature polypectomy snare surgical method according to a preferred embodiment of the invention;
Fig.5 is a structure diagram of a low temperature polypectomy snare surgical device according to a preferred embodiment of the invention; and
Fig.6-Fig.8 are partially enlarged or cross section schematic diagrams of a low temperature polypectomy snare surgical device according to a preferred embodiment of the invention.

### Detailed Description of the Embodiments

The embodiments of the present invention are described below by specific embodiments, and those skilled in the art can easily understand other advantages and effects of the present invention from the contents disclosed by the present specification.

Exemplary embodiments of the present invention will now be described with reference to the drawings, however, the present invention can be embodied in many different forms and is not limited to the embodiments described herein. These embodiments are provided to completely disclose the present invention in detail and to fully communicate the scope of the present invention to those skilled in the art to which the present invention belongs. The terms used in the exemplary embodiments shown in the drawings are not intended to limit the present invention. In the drawings, the same units/elements are marked by the same reference signs.

Unless otherwise stated, the terms (including technical and scientific terms) used herein have the ordinary meanings understood by those skilled in the art. In addition, it should be understood that the terms defined by commonly used dictionaries should be understood as having meanings consistent with the contexts of the relevant field, and should not be construed as idealized or overly formal meanings.

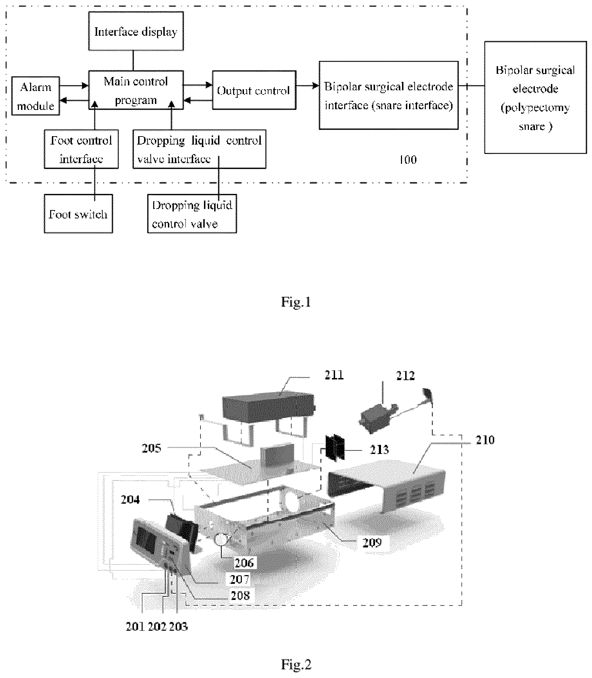

Fig.1 is a functional schematic diagram of a plasma therapeutic apparatus 100 according to a preferred embodiment of the present invention. The plasma therapeutic apparatus 100 can be applied to the snare cutting, ablation, coagulation and hemostasis of such as polyps or prominent tumor sites in the digestive tract. In addition, the plasma therapeutic apparatus 100 further can be applied to the snare cutting, ablation, coagulation and hemostasis of soft tissues in joint, spine, skin, ear-nose-throat and other surgeries. The plasma therapeutic apparatus 100 of the present application is used within 24 hours, belongs to temporary contact if being classified according to contact time, belongs to an external access instrument (with tissues/bones/dentine) if being classified according to the nature of the contacted human body, and belongs to active medical equipment if being classified according to the structural characteristics of the medical equipment. An accessory, namely, a bipolar surgical electrode (snare) head of the plasma therapeutic apparatus 100 is a disposable sterile product.

The plasma therapeutic apparatus 100 adopts a bipolar solution and works at a frequency of 110 kHz. The plasma therapeutic apparatus 100 realizes the snare cutting, ablation, coagulation and hemostasis of soft tissues in the surgeries such as the ear-nose-throat surgery. During the work, the plasma therapeutic apparatus 100 employs normal saline as conductive liquid and forms a thin layer while activating an emission electrode and a loop electrode. When the plasma therapeutic apparatus 100 gives sufficient energy (voltage), the normal saline is converted into a gas layer (plasma layer) composed of energized charged particles. That is, the plasma therapeutic apparatus 100 utilizes energy to excite a conductive medium (e.g., normal saline) to generate plasma, and relies on the energy of the plasma to break molecular bonds of the tissue. The energy of the plasma directly cleaves biomacromolecules such as proteins into gases such as O², CO², N² and the like, thereby completing vaporization snare cutting of the tissue. When a low voltage is applied to a working knife head of the plasma therapeutic apparatus 100, an electric field is lower than the threshold requirement for generating the plasma layer, and resistance heat of the tissue is generated, thereby achieving ablation, coagulation and hemostasis of the tissue.

As shown in Fig.1, the functional system structure of the plasma therapeutic apparatus 100 includes: a main control program, an alarm unit, an interface unit, an output control unit, a bipolar surgical electrode (snare) interface, a bipolar surgical electrode (snare), a foot switch, a foot control interface, a dropping liquid control valve and a dropping liquid control valve interface. The main control program, the alarm unit, the interface unit, the output control unit, the bipolar surgical electrode interface, the foot control interface and the dropping liquid control valve interface belong to software parts of the plasma therapeutic apparatus 100. The functions of some components of the plasma therapeutic apparatus 100 are introduced, as shown in Table 1.

**Table 1 Function introduction of some components**

| Module name | Function | Associated module | Interface relationship | User interface |
|---|---|---|---|---|
| Main control program | Having a self-inspection function, monitoring the bipolar surgical electrode and the foot switch connection situation, and calculating the impedance and the actual power | All modules | Signal and energy Input and output interface | Presence |
| Alarm unit | Detecting an alarm signal and alarmig via voice prompt, character prompt and indicator lamp display | Main control program Interface unit | Input and output via IO | Presence |
| Interface unit | Displaying values, alarms and the like | Main control program | Input and output via IO | Presence |
| Output control unit | Accepting the power of the main control program for the bipolar surgical electrode interface, and transmitting the impedance of the bipolar surgical electrode interface to the | Main control program Interface unit | Energy output | Presence |
| | main control program | | | |
| Foot control interface | Distributing a key message | Main control program Interface unit | Signal input | Presence |
| Dropping liquid control valve interface | Distributing a key message | Main control program | Signal input | Absence |

Preferably, the foot switch can control the working mode of the plasma therapeutic apparatus 100. The working mode of the plasma therapeutic apparatus 100 is divided into a snare-cutting mode and a coagulation mode. The waterproof level of the foot switch is the waterproof level standard IPX8, and the foot switch is an electric foot switch.

Preferably, a yellow pedal of the foot switch corresponds to the snare-cutting mode, and gear levels of the snare-cutting mode include first to ninth gears. That is, when the yellow pedal of the foot switch is stepped on, the plasma therapeutic apparatus 100 enters the snare cutting mode. A gear adjustment way of the snare-cutting mode is performing adjustment by a black button on the foot switch (or a yellow button on a host panel is manually adjusted) in the state that the plasma therapeutic apparatus is adjusted to the snare-cutting mode. The snare-cutting gear can be selected from any of the first to ninth gears. The higher the gear is, the greater the output voltage is. In the snare-cutting mode, the output voltages of first to ninth gears are as shown in Table 2:

**Table 2 Output gears in the snare-cutting mode**

| | Gear | Rated output voltage (Vrms) | Output voltage error |
|---|---|---|---|
| Output voltage in the snare-cutt ing mode | 1 | 100 | ±10% |
| | 2 | 125 | ±10% |
| | 3 | 150 | ±10% |
| | 4 | 175 | ±10% |
| | 5 | 200 | ±10% |
| | 6 | 225 | ±10% |
| | 7 | 250 | ±10% |
| | 8 | 275 | ±10% |
| | 9 | 300 | ±10% |

Preferably, a blue pedal of the foot switch corresponds to the coagulation mode, and the gear levels of the coagulation mode include first to fifth gears. That is, when the blue pedal of the foot switch is stepped on, the plasma therapeutic apparatus 100 enters the coagulation mode. The gear adjustment way of the coagulation mode is performing adjustment by the black button on the blue pedal (or a blue button on the host panel is manually adjusted) in the state that the plasma therapeutic apparatus is adjusted to the coagulation mode (the snare-cutting mode and the coagulation mode can be switched by pressing a mode button). The coagulation gear can be selected from any of the first to fifth gears when the black button is stepped on, wherein the higher the gear is, the greater the output voltage is. When coagulation is required in clinical use, the blue penal is stepped on to perform the coagulation. The output voltages of first to fifth gears in the coagulation mode are as shown in Table 3:

**Table 3 Output gears in the coagulation mode**

| | Gear | Rated output voltage (Vrms) | Output voltage error |
|---|---|---|---|
| Output voltage in the coagulati on mode | 1 | 60 | ±10% |
| | 2 | 65 | ±10% |
| | 3 | 70 | ±10% |
| | 4 | 75 | ±10% |
| | 5 | 80 | ±10% |

Preferably, the foot control interface is configured to receive a control instruction of the foot switch and forward the control instruction to the main control program. The control instruction is a two-tuples <mode, power>. The modes include the snare-cutting mode and the coagulation mode. In the snare-cutting mode, the power includes 9 gears, and in the coagulation mode, the power includes 5 gears.

Preferably, the main control program parses the control instruction, generates a first mode instruction when the control instruction indicates a first mode, calculates the output power in the first mode according to the current impedance and the control instruction, and sends the first mode instruction and a first voltage indication associated with the output power in the first mode to the output control unit. The initial current impedance is zero, that is, when the plasma therapeutic apparatus 100 is powered on for operation, the default current impedance is zero. The main control program parses the control instruction, generates a second mode instruction when the control instruction indicates a second mode, calculates the output power in the second mode according to the current impedance and the control instruction, and sends the second mode instruction and a second voltage indication associated with the output power in the second mode to the output control unit. The current impedance includes high impedance, medium impedance, and low impedance (zero impedance is low impedance). Preferably, the calculating the output power in the first mode according to the current impedance and the control instruction includes: if the current impedance is the high impedance and the control instruction indicates the second gear in the first mode, setting the output power in the first mode as the fourth gear; if the current impedance is the medium impedance and the control instruction indicates the second gear in the first mode, setting the output power in the first mode as the third gear; and if the current impedance is the low impedance and the control instruction indicates the second gear in the first mode, setting the output power in the first mode as the second gear. Preferably, the calculating the output power in the second mode according to the current impedance and the control instruction includes: if the current impedance is the high impedance and the control instruction indicates the second gear in the second mode, setting the output power in the second mode as the fourth gear; if the current impedance is the medium impedance and the control instruction indicates the second gear in the second mode, setting the output power in the second mode as the third gear; and if the current impedance is the low impedance and the control instruction indicates the second gear in the second mode, setting the output power in the second mode as the second gear. Preferably, when the calculated output power exceeds the highest gear in the first mode or the second mode, the highest gear is used as the actual output power.

Preferably, the output control unit is configured to receive the first mode instruction and the first voltage indication from the main control program, forward the first mode instruction and the first voltage indication to the bipolar surgical electrode interface, receive the current impedance of a target contact end from the bipolar surgical electrode interface and send the current impedance to the main control program. The output control unit receives the second mode instruction and the second voltage indication from the main control program and forwards the second mode instruction and the second voltage indication to the bipolar surgical electrode interface.

Preferably, the bipolar surgical electrode interface is configured to receive a power indication of the main control program, send the power indication to the bipolar surgical electrode, measure the real-time impedance of the bipolar surgical electrode and transmit the real-time impedance to the main control program through the output control unit.

Preferably, the bipolar surgical electrode enters the first mode in response to the reception of the first mode instruction and the first voltage indication from the bipolar surgical electrode interface, in which mode circuit activation is performed between an emission electrode and a loop electrode at the target contact end of the bipolar surgical electrode via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma. The bipolar surgical electrode enters the second mode in response to the reception of the second instruction and the second voltage indication from the bipolar surgical electrode interface, in which a second voltage is applied to maintain the target contact end of the bipolar surgical electrode at a second temperature so as to perform ablation and coagulation of the target object.

Preferably, the alarm unit is configured to perform alarm via voice prompt, character prompt and/or indicator lamp display when an alarm signal is received. The bipolar surgical electrode sends the alarm signal to the main control program after detecting an operation fault, and the main control program sends the alarm signal to the alarm unit.

Preferably, the interface unit is configured to display the operation state of the low temperature polypectomy snare surgical system in real time.

Preferably, the dropping liquid control valve is configured to input the conductive medium to the bipolar surgical electrode based on a conductive medium input instruction of the main control program, wherein the bipolar surgical electrode measures a current residual amount of the conductive medium in real time and sends the current residual amount to the main control program, the main control program determines whether to generate the conductive medium input instruction based on the current residual amount and sends the conductive medium input instruction to the dropping liquid control valve after determining to generate the conductive medium input instruction. Preferably, the dropping liquid control valve interface is configured to achieve two-way communication between the dropping liquid control valve and the main control program.

Preferably, the emission electrode at the catheter of the tail end of the bipolar surgical electrode, the plasma layer, the loop electrode and the target contact end form a loop. In the snare-cutting mode, the working temperature of the bipolar surgical electrode is 35 to 40°C, while the working temperature of the conventional electrosurgical knife is 350 to 700°C. The thermal penetration distance of the bipolar surgical electrode is less than the thermal penetration distance of the conventional electrosurgical knife, wherein the thermal penetration distance in the snare-cutting mode is less than or equal to 150 microns, the thermal penetration distance in the coagulation mode is less than or equal to 200 microns, and the thermal penetration distance of the conventional electrosurgical knife is greater than 9000 microns.

The working principle of the plasma therapeutic apparatus 100 is the plasma low temperature ablation. Energy is generated by a bipolar knife head to convert the normal saline into a thin layer of plasma so as to dissociate molecular bonds constituting cellular components in a target tissue, thereby causing coagulative necrosis of the tissue to form an ablation or snare-cutting effect. Due to the work at a relatively low temperature, the thermal damage to the surrounding tissues is minimized compared with the conventional high-frequency snare-cutting electric knives. At a working temperature of about 35°C, the volume of the target tissue can be reduced, microvessels in the target tissue are closed, and the lesion is excised. Due to the low temperature and tissue shrinking and ablation characteristics, it has the advantages of shortening the postoperative recovery time, relieving the postoperative pain and reducing the cost of surgical treatment compared with the commonly used monopolar electric knives. The temperature comparison between the plasma therapeutic apparatus and the common high-frequency snare-cutting electric knife is shown in Table 4.

**Table 4**

| | Snare-cutting temperature | Coagulation temperature |
|---|---|---|
| Plasma therapeutic apparatus | 35°C-40°C | 40°C-70°C |
| High-frequency snare-cutting electric knife (Ellman high frequency surgical system) | Mean 126.3±15.47°C maximum value 149.9°C | Mean 126.3±15.47°C maximum value 149.9°C |

When the plasma therapeutic apparatus is working, the ambient temperature of the knife head is lower than 70°C (see a tissue thermal damage report of in vitro experimental study for details), the working temperature is lower than that (a high temperature of 100-150°C) of the conventional common high-frequency snare-cutting electric knife, although the treatment temperature of the low-temperature plasma knife is still a high temperature relative to human tissues, after the tissue cells are affected by the temperature, tissue protein degeneration is also generated by the snare cutting performed by the electric knife, and the thermal damage is also generated to the tissues after a period of time. The degeneration and necrosis of the tissue cells is a gradual development process, therefore, after some patients undergo low-temperature plasma surgery, and the reactions of swelling in the surgical area, postoperative pain and the like are not lighter than those of the high-frequency snare-cutting electric knife. The thermal damage depth comparison between the plasma therapeutic apparatus and the common high-frequency snare-cutting electric knife is shown in Table 5 below:

**Table 5**

| | Thermal damage depth during snare-cutting | Thermal damage depth during coagulation |
|---|---|---|
| Plasma therapeutic apparatus | Average value 150 microns | Average value 200 microns |
| High-frequency snare-cutting electric knife | 1.23±0.24 mm | 1.37±0.26 mm |

Due to the difference in time of each surgery, the plasma therapeutic apparatus selects the maximum surgical time in the tissue thermal damage report of in vitro experimental study, and thus the thermal damage depths of the plasma therapeutic apparatus with the maximum surgical time and the normally used high-frequency snare-cutting electric knife can be obtained by comparison. Therefore, the thermal damage depth of the normally used plasma therapeutic apparatus should be lower than the thermal damage depth of the high-frequency snare-cutting electric knife.

Fig.2 is a schematic diagram of main parts of a plasma therapeutic apparatus 200 according to a preferred embodiment of the invention. As shown in Fig.2, the main parts of the plasma therapeutic apparatus 200 include: a bipolar surgical electrode interface 201, a dropping liquid control valve interface 202, a foot switch interface 203, a display screen 204, a main board 205, a horn 206, a front panel 207, a fault alarm light 208, a lower die 209, an upper die 210, a power module 211, a dropping liquid control valve 212 and a fan 213.

Preferably, the foot switch interface 203 is configured to receive an instruction of the foot switch, and the foot switch can control the working mode of the plasma therapeutic apparatus 200. The working mode of the plasma therapeutic apparatus 200 is divided into a snare-cutting mode and a coagulation mode. The waterproof level of the foot switch is the waterproof level standard IPX8, and the foot switch is an electric foot switch.

Preferably, a yellow pedal of the foot switch corresponds to the snare-cutting mode, and the gear levels of the snare-cutting mode include first to ninth gears. That is, when the yellow pedal of the foot switch is stepped on, the plasma therapeutic apparatus 200 enters the snare-cutting mode. A gear adjustment way of the snare-cutting mode is performing adjustment by a black button on the foot switch (or a yellow button on a host panel is manually adjusted) in the state that the plasma therapeutic apparatus is adjusted to the snare-cutting mode. The snare-cutting gear can be selected from any of the first to ninth gears. The higher the gear is, the greater the output voltage is.

Preferably, a blue pedal of the foot switch corresponds to the coagulation mode, and the gear levels of the coagulation mode include first to fifth gears. That is, when the blue pedal of the foot switch is stepped on, the plasma therapeutic apparatus 200 enters the coagulation mode. The gear adjustment way of the coagulation mode is performing adjustment by the black button on the blue pedal (or a blue button on the host panel is manually adjusted) in the state that the plasma therapeutic apparatus is adjusted to the coagulation mode (the snare-cutting mode and the coagulation mode can be switched by pressing a mode button). The coagulation gear can be selected from any of the first to fifth gears when the black button is stepped on, wherein the higher the gear is, the greater the output voltage is. When coagulation is required in clinical use, the blue penal is stepped on to perform the coagulation.

Preferably, the foot switch interface 203 is configured to receive a control instruction of the foot switch and forward the control instruction to a main control program. The control instruction is a two-tuples <mode, power>. The modes include the snare-cutting mode and the coagulation mode. In the snare-cutting mode, the power includes 9 gears, and in the coagulation mode, the power includes 5 gears.

Preferably, the main board 205 is configured to accommodate firmware, and the firmware stores the main control program. The main control program parses the control instruction, generates a first mode instruction when the control instruction indicates a first mode, calculates the output power in the first mode according to the current impedance and the control instruction, and sends the first mode instruction and a first voltage indication associated with the output power in the first mode to an output control unit. The initial current impedance is zero, that is, when the plasma therapeutic apparatus 200 is powered on for operation, the default current impedance is zero. The main control program parses the control instruction, generates a second mode instruction when the control instruction indicates a second mode, and calculates the output power in the second mode according to the current impedance and the control instruction, and sends the second mode instruction and a second voltage indication associated with the output power in the second mode to the output control unit. The current impedance includes high impedance, medium impedance, and low impedance (zero impedance is low impedance). Preferably, the calculating the output power in the first mode according to the current impedance and the control instruction includes; if the current impedance is the high impedance and the control instruction indicates the second gear in the first mode, setting the output power in the first mode as the fourth gear; if the current impedance is the medium impedance and the control instruction indicates the second gear in the first mode, setting the output power in the first mode as the third gear; and if the current impedance is the low impedance and the control instruction indicates the second gear in the first mode, setting the output power in the first mode as the second gear. Preferably, the calculating the output power in the second mode according to the current impedance and the control instruction includes: if the current impedance is the high impedance and the control instruction indicates the second gear in the second mode, setting the output power in the second mode as the fourth gear; if the current impedance is the medium impedance and the control instruction indicates the second gear in the second mode, setting the output power in the second mode as the third gear; and if the current impedance is the low impedance and the control instruction indicates the second gear in the second mode, setting the output power in the second mode as the second gear. Preferably, when the calculated output power exceeds the highest gear in the first mode or the second mode, the highest gear is used as the actual output power

Preferably, the output control unit (not shown in Fig.2) is configured to receive the first mode instruction and the first voltage indication from the main control program, forward the first mode instruction and the first voltage indication to the bipolar surgical electrode interface 201, receive the current impedance of a target contact end from the bipolar surgical electrode interface 201 and send the current impedance to the main control program. The output control unit receives the second mode instruction and the second voltage indication from the main control program and forwards the second mode instruction and the second voltage indication to the bipolar surgical electrode interface 201.

Preferably, the bipolar surgical electrode interface 201 is configured to receive a power indication of the main control program, send the power indication to the bipolar surgical electrode, measure the real-time impedance of the bipolar surgical electrode and transmit the real-time impedance to the main control program through the output control unit.

Preferably, the bipolar surgical electrode (not shown in the figure) enters the first mode in response to the reception of the first mode instruction and the first voltage indication from the bipolar surgical electrode interface 201, in which mode circuit activation is performed between an emission electrode and a loop electrode at the target contact end of the bipolar surgical electrode via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma. The bipolar surgical electrode enters the second mode in response to the reception of the second instruction and the second voltage indication from the bipolar surgical electrode interface 201, in which mode a second voltage is applied to maintain the target contact end of the bipolar surgical electrode at a second temperature so as to perform ablation and coagulation of the target object.

Preferably, the fault alarm light 208 is configured to perform alarm via indicator lamp display when an alarm signal is received. The bipolar surgical electrode sends the alarm signal to the main control program after detecting an operation fault, and the main control program sends the alarm signal to the fault alarm light 208. The horn 206 is configured to perform alarm via voice when receiving the alarm signal. The bipolar surgical electrode sends the alarm signal to the main control program after detecting the operation fault, and the main control program sends the alarm signal to the horn 206. Preferably, the display screen 204 is configured to display the operation state of the low temperature polypectomy snare surgical system in real time.

Preferably, the dropping liquid control valve 212 is configured to input the conductive medium to the bipolar surgical electrode based on a conductive medium input instruction of the main control program, wherein the bipolar surgical electrode measures a current residual amount of the conductive medium in real time and sends the current residual amount to the main control program, the main control program determines whether to generate the conductive medium input instruction based on the current residual amount and sends the conductive medium input instruction to the dropping liquid control valve 212 after determining to generate the conductive medium input instruction. Preferably, the dropping liquid control valve interface 202 is configured to achieve the two-way communication between the dropping liquid control valve 212 and the main control program.

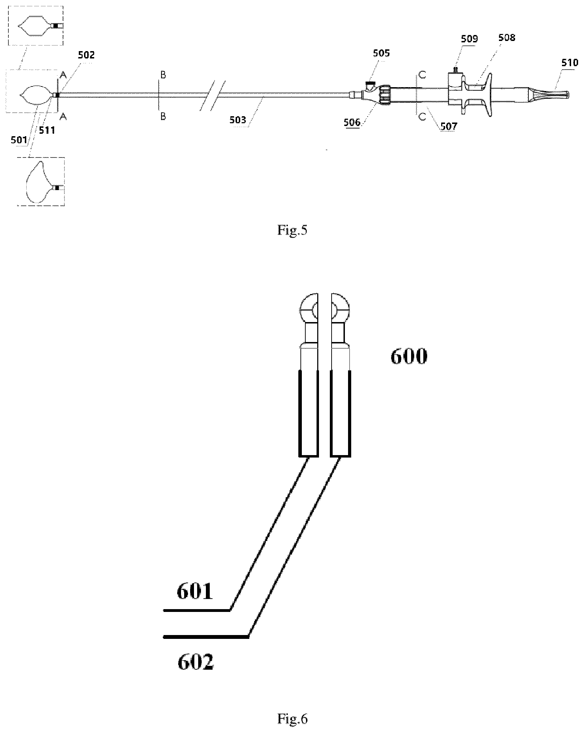

Preferably, the present application employs a dual-mode liquid outlet: 1. a titration mode, that is, a mode of supplying drop by drop just like the infusion of an infusion bottle; and 2. a continuous supply mode, that is, a mode of continuously supplying liquid flow. The bipolar surgical electrode interface (snare interface) is connected with a bipolar electrode socket connector (snare connector) of Fig.5 as described below through a patching cord, and the dropping liquid control valve interface is connected with a liquid injection cavity of Fig.5 through a connecting tube. The foot switch port is connected with an external foot pedal through a connecting cable for controlling the supply and disconnection of energy and dropping liquid. When the foot pedal is stepped down, the energy and the dropping liquid are supplied; and when the foot pedal is released, the energy and the dropping liquid are disconnected.

Preferably, the emission electrode at the catheter of the tail end of the bipolar surgical electrode, the plasma layer, the loop electrode and the target contact end form a loop. In the snare-cutting mode, the working temperature of the bipolar surgical electrode is 35 to 40°C, while the working temperature of the conventional electrosurgical knife is 350 to 700°C. The thermal penetration distance of the bipolar surgical electrode is less than the thermal penetration distance of the conventional electrosurgical knife, wherein the thermal penetration distance in the snare-cutting mode is less than or equal to 150 microns, the thermal penetration distance in the coagulation mode is less than or equal to 200 microns, and the thermal penetration distance of the conventional electrosurgical knife is greater than 9000 microns.

Preferably, the upper die 210 and the lower die 209 are combined to protect the main board. The fan 213 is configured to perform heat dissipation, and the power module 211 is configured to supply power to the plasma therapeutic apparatus 200. The front panel 207 is configured to perform data display and operation control.

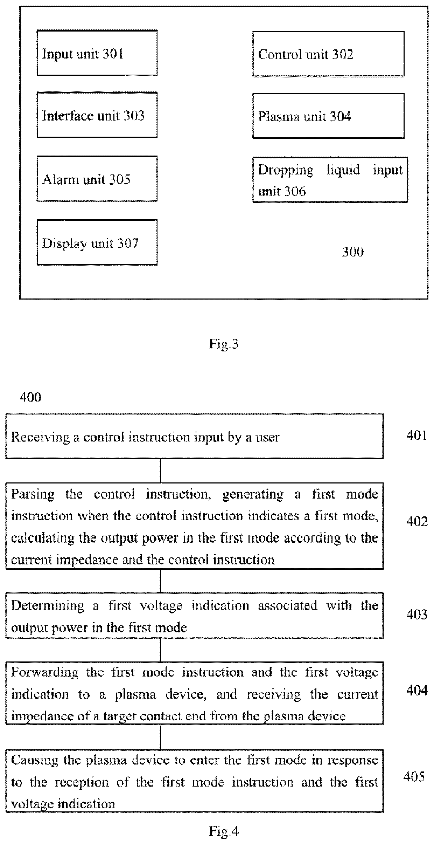

Fig.3 is a structure diagram of a low temperature polypectomy snare surgical system 300 according to a preferred embodiment of the invention. The low temperature polypectomy snare surgical system 300 can be applied to the snare cutting, ablation, coagulation and hemostasis of such as polyps or prominent tumor sites in the digestive tract. In addition, the low temperature polypectomy snare surgical system 300 can also be applied to the snare cutting, ablation, coagulation and hemostasis of soft tissues in joint, spine, skin, ear-nose-throat and other surgeries. The low temperature polypectomy snare surgical system 300 of the present application is used within 24 hours, belongs to temporary contact if being classified according to contact time, belongs to an external access instrument (with tissues/bones/dentine) if being classified according to the nature of the contacted human body, and belongs to active medical equipment if being classified according to the structural characteristics of the medical equipment.

The low temperature polypectomy snare surgical system 300 adopts a bipolar solution and works at a frequency of 110 kHz. The low temperature polypectomy snare surgical system 300 realizes the snare cutting, ablation, coagulation and hemostasis of soft tissues in the surgeries such as the ear-nose-throat surgery. During the work, the low temperature polypectomy snare surgical system 300 employs normal saline as conductive liquid and forms a thin layer while activating an emission electrode and a loop electrode. When the low temperature polypectomy snare surgical system 300 gives sufficient energy (voltage), the normal saline is converted into a gas layer (plasma layer) composed of energized charged particles. That is, the low temperature polypectomy snare surgical system 300 utilizes energy to excite a conductive medium (e.g., normal saline) to generate plasma, and relies on the energy of the plasma to break molecular bonds of the tissue. The energy of the plasma directly cleaves biomacromolecules such as proteins into gases such as O², CO², N² and the like, thereby completing the vaporization snare cutting of the tissue. When a low voltage is applied to a working knife head of the plasma therapeutic apparatus 100, an electric field is lower than the threshold requirement for generating the plasma layer, and resistance heat of the tissue is generated, thereby achieving ablation, coagulation and hemostasis of the tissue.

As shown in Fig.3, the low temperature polypectomy snare surgical system 300 includes: an input unit 301, a control unit 302, an interface unit 303, a plasma unit 304, an alarm unit 305, a dropping liquid input unit 306 and a display unit 307. Preferably, for example, the input unit 301 is a foot switch, and the foot switch can control the working mode of the low temperature polypectomy snare surgical system 300. The working mode of the low temperature polypectomy snare surgical system 300 is divided into a snare-cutting mode and a coagulation mode. The waterproof level of the foot switch is the waterproof level standard IPX8, and the foot switch is an electric foot switch. Preferably, a yellow pedal of the foot switch corresponds to the snare-cutting mode, and the gear levels of the snare-cutting mode include first to ninth gears. That is, when the yellow pedal of the foot switch is stepped on, the low temperature polypectomy snare surgical system 300 enters the snare cutting mode. A gear adjustment way of the snare-cutting mode is performing adjustment by a black button on the foot switch (or a yellow button on a host panel is manually adjusted) in the state that the plasma therapeutic apparatus is adjusted to the snare-cutting mode. The snare-cutting gear can be selected from any of the first to ninth gears. The higher the gear is, the greater the output voltage is.

Preferably, a blue pedal of the foot switch corresponds to the coagulation mode, and the gear levels of the coagulation mode include first to fifth gears. That is, when the blue pedal of the foot switch is stepped on, the low temperature polypectomy snare surgical system 300 enters the coagulation mode. The gear adjustment way of the coagulation mode is performing adjustment by the black button on the blue pedal (or a blue button on the host panel is manually adjusted) in the state that the plasma therapeutic apparatus is adjusted to the coagulation mode (the snare-cutting mode and the coagulation mode can be switched by pressing a mode button). The coagulation gear can be selected from any of the first to fifth gears when the black button is stepped on, wherein the higher the gear is, the greater the output voltage is. When coagulation is required in clinical use, the blue penal is stepped on to perform the coagulation.

Preferably, a foot control interface is configured to receive a control instruction of the foot switch and forward the control instruction to a main control program. The control instruction is a two-tuples <mode, power>. The modes include the snare-cutting mode and the coagulation mode. In the snare-cutting mode, the power includes 9 gears, and in the coagulation mode, the power includes 5 gears.

Preferably, the control unit 302 parses the control instruction, generates a first mode instruction when the control instruction indicates a first mode, calculates the output power in the first mode according to the current impedance and the control instruction, and sends the first mode instruction and a first voltage indication associated with the output power in the first mode to the interface unit 303. The initial current impedance is zero, that is, when the low temperature polypectomy snare surgical system 300 is powered on for operation, the default current impedance is zero. The control unit 302 parses the control instruction, generates a second mode instruction when the control instruction indicates a second mode, and calculates the output power in the second mode according to the current impedance and the control instruction, and sends the second mode instruction and a second voltage indication associated with the output power in the second mode to the interface unit 303. The current impedance includes high impedance, medium impedance, and low impedance (zero impedance is low impedance). Preferably, the calculating the output power in the first mode according to the current impedance and the control instruction includes; if the current impedance is the high impedance and the control instruction indicates the second gear in the first mode, setting the output power in the first mode as the fourth gear; if the current impedance is the medium impedance and the control instruction indicates the second gear in the first mode, setting the output power in the first mode as the third gear; and if the current impedance is the low impedance and the control instruction indicates the second gear in the first mode, setting the output power in the first mode as the second gear. Preferably, the calculating the output power in the second mode according to the current impedance and the control instruction includes: if the current impedance is the high impedance and the control instruction indicates the second gear in the second mode, setting the output power in the second mode as the fourth gear; if the current impedance is the medium impedance and the control instruction indicates the second gear in the second mode, setting the output power in the second mode as the third gear; and if the current impedance is the low impedance and the control instruction indicates the second gear in the second mode, setting the output power in the second mode as the second gear. Preferably, when the calculated output power exceeds the highest gear in the first mode or the second mode, the highest gear is used as the actual output power

Preferably, the interface unit 303 is configured to receive the first mode instruction and the first voltage indication from the control unit 302, forward the first mode instruction and the first voltage indication to the plasma unit 304, receive the current impedance of a target contact end from the plasma unit 304 and send the current impedance to the control unit 302. The interface unit 303 receives the second mode instruction and the second voltage indication from the control unit 302 and forwards the second mode instruction and the second voltage indication to the plasma unit 304. Preferably, the interface unit 303 is configured to receive a power indication of the control unit 302, send the power indication to the plasma unit 304, measure the real-time impedance of the plasma unit 304 and transmit the real-time impedance to the control unit 302 through the interface unit 303. Preferably, the plasma unit 304 enters the first mode in response to the reception of the first mode instruction and the first voltage indication from the plasma unit 304, in which circuit activation is performed between an emission electrode and a loop electrode at the target contact end of the plasma unit 304 via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma. The plasma unit 304 enters the second mode in response to the reception of the second instruction and the second voltage indication from the plasma unit 304, in which mode a second voltage is applied to maintain the target contact end of the plasma unit 304 at a second temperature so as to perform ablation and coagulation of the target object.

Preferably, the alarm unit 305 is configured to perform alarm via voice prompt, character prompt and/or indicator lamp display when an alarm signal is received. The plasma unit 304 sends the alarm signal to the control unit 302 after detecting an operation fault, and the control unit 302 sends the alarm signal to the alarm unit 305.

Preferably, the dropping liquid input unit 306 is configured to input the conductive medium to the plasma unit 304 based on a conductive medium input instruction of the control unit 302, wherein the plasma unit 304 measures a current residual amount of the conductive medium in real time and sends the current residual amount to the control unit 302, the control unit 302 determines whether to generate the conductive medium input instruction based on the current residual amount and sends the conductive medium input instruction to the dropping liquid input unit 306 after determining to generate the conductive medium input instruction.

Preferably, the emission electrode at the catheter of the tail end of the plasma unit 304, the plasma layer, the loop electrode and the target contact end form a loop. In the snare-cutting mode, the working temperature of the plasma unit 304 is 35 to 40°C, while the working temperature of the conventional electrosurgical knife is 350 to 700°C. The thermal penetration distance of the plasma unit 304 is less than the thermal penetration distance of the conventional electrosurgical knife, wherein the thermal penetration distance in the snare-cutting mode is less than or equal to 150 microns, the thermal penetration distance in the coagulation mode is less than or equal to 200 microns, and the thermal penetration distance of the conventional electrosurgical knife is greater than 9000 microns.

Preferably, the display unit 307 is configured to display an operation state of the low temperature polypectomy snare surgical system in real time. The working principle of the low temperature polypectomy snare surgical system 300 is the plasma low temperature ablation. Energy is generated by a bipolar knife head to convert the normal saline into a thin layer of plasma so as to dissociate molecular bonds constituting cellular components in a target tissue, thereby causing coagulative necrosis of the tissue to form an ablation or snare cutting effect. Due to the work at a relatively low temperature, the thermal damage to the surrounding tissues is minimized compared with the conventional high-frequency snare-cutting electric knives. At a working temperature of about 35°C, the volume of the target tissue can be reduced, microvessels in the target tissue are closed, and the lesion is excised. Due to the low temperature and tissue shrinking and ablation characteristics, it has the advantages of shortening the postoperative recovery time, relieving the postoperative pain and reducing the cost of surgical treatment compared with the commonly used monopolar electric knives. When the low temperature polypectomy snare surgical system 300 is working, the ambient temperature of the knife head is lower than 70°C (see a tissue thermal damage report of in vitro experimental study), the working temperature is lower than that (a high temperature of 100-150°C) of the conventional common high-frequency snare-cutting electric knife, although the treatment temperature of the low-temperature plasma knife is still a high temperature relative to human tissues, after the tissue cells are affected by the temperature, tissue protein degeneration is also generated by the snare cutting performed by the electric knife, and the thermal damage is also generated to the tissues after a period of time. The degenerative necrosis of the tissue cells is a gradual development process, therefore, after some patients are subjected to low-temperature plasma surgeries, and the reactions of swelling in the operation area, postoperative pain and the like are not lighter than those of the high-frequency snare-cutting electric knife. Due to the difference in the time of each surgery, the plasma therapeutic apparatus selects the maximum surgical time in the tissue thermal damage report of in vitro experimental study, and thus the thermal damage depths of the plasma therapeutic apparatus with the maximum surgical time and the normally used high-frequency snare-cutting electric knife can be obtained by comparison. Therefore, the thermal damage depth of the normally used plasma therapeutic apparatus should be lower than the thermal damage depth of the high-frequency snare-cutting electric knife.

Fig.4 is a flowchart of a low temperature polypectomy snare surgical method 400 according to a preferred embodiment of the invention. As shown in Fig.4, the method 400 is started from step 401. In step 401, a control instruction input by a user is received.

In step 402, the control instruction is parsed, a first mode instruction is generated when the control instruction indicates a first mode, and the output power in the first mode is calculated according to the current impedance and the control instruction.

In step 403, a first voltage indication associated with the output power in the first mode is determined.

In step 404, the first mode instruction and the first voltage indication are forwarded to a plasma device, and the current impedance of a target contact end is received from the plasma device.

In step 405, the plasma device is caused to enter the first mode in response to the reception of the first mode instruction and the first voltage indication, in which mode circuit activation is performed between an emission electrode and a loop electrode at the target contact end of the plasma unit via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma;
wherein the emission electrode, the plasma layer, the loop electrode and the target contact end form a loop.

The low temperature polypectomy snare surgical method further includes: parsing the control instruction, generating a second mode instruction when the control instruction indicates a second mode, calculating the output power in the second mode according to the current impedance and the control instruction, and determining a second voltage indication associated with the output power in the second mode; forwarding the second mode instruction and the second voltage indication to the plasma device; and causing the plasma device to enter the second mode in response to the reception of the second mode instruction and the second voltage indication, in which mode a second voltage is applied to maintain the target contact end of the plasma unit at a second temperature so as to perform ablation and coagulation of the target object.

The low temperature polypectomy snare surgical method further includes: performing alarm via voice prompt, character prompt and/or indicator lamp display when an alarm signal is received; and generating the alarm signal after detecting an operation fault.

The user generates the control instruction by operating a pedal type input device, wherein the control instruction is a two-tuples <mode, power>.

The low temperature polypectomy snare surgical method further includes: inputting the conductive medium to the plasma device based on a conductive medium input instruction, wherein the plasma device measures a current residual amount of the conductive medium in real time and determines whether to generate the conductive medium input instruction based on the current residual amount. The method 400 includes: displaying an operation state of the plasma device in real time.

Fig.5 is a structural schematic diagram of a low temperature polypectomy snare surgical device according to a preferred embodiment of the invention. As shown in Fig.5, the low temperature polypectomy snare surgical device includes: an emission electrode (snare head) 501, a loop electrode (circular sleeve) 502, a tube sheath 503, an injection cavity interface 505, a pull rod cap 506, a cushion block 507, a slide block (with a socket hole) 508, a socket Pin 509, and a pull rod 510. Preferably, the emission electrode (snare head) 501 and the loop electrode (circular sleeve) 502 are introduced through the same catheter and form a conductive loop on a target object. The emission electrode (snare head) 501 receives a first input voltage generated by a high frequency generator via the socket Pin 509, a first voltage is applied between the emission electrode (snare head) 501 and the loop electrode (circular sleeve) 502, so that the conductive medium reach a first temperature and is converted into a plasma layer, thereby exciting the conductive medium with electrical energy to generate plasma and performing vaporization snare cutting on the target object based on the radio frequency energy of the plasma. The tube sheath 503 is configured to provide an outer layer coating function. The injection cavity interface 505 inputs the liquid to a liquid input unit based on a liquid input instruction, wherein the liquid input unit measures the current residual amount of the liquid in real time and sends the current residual amount to a control unit, and the control unit determines whether to generate the liquid input instruction based on the current residual amount and sends the liquid input instruction to a dropping liquid input unit after determining to generate the liquid input instruction. The injection cavity interface 505 is an annular cavity located at the outside of an emission electrode conducting wire (snare head conducting wire) described later.

The pull rod 510 is configured to enable an operator to provide a supporting force by holding the pull rod 510. The socket Pin 509 is connected with the high frequency generator through a high frequency cable to receive the first input voltage generated by the high frequency generator. The socket Pin 509 receives a second input voltage generated by the high frequency generator and transmits the second input voltage to the emission electrode, a second voltage is applied between the emission electrode 501 and the loop electrode 502 so as to maintain the target object at a second temperature, thereby promoting ablation and coagulation of the target object. The liquid input unit (not shown in Fig.5) inputs liquid to the target object in response to the liquid input signal to form the thin layer of the conductive medium between the emission electrode and the loop electrode. The liquid input unit performs liquid input by one of a titration mode and a continuous liquid supply mode.

The material of the emission electrode (snare head) 501 is stainless steel 304, the material of the loop electrode (circular sleeve) 502 is the stainless steel 304, the material of the tube sheath 503 is polytetrafluoroethylene PTFE, the material of the injection cavity interface 505 is acrylonitrile-butadiene-slyrene copolymer ABS, the material of the pull rod cap 506 is ABS, the material of the cushion block 507 is ABS, the material of the slide block (with socket hole) ABS, the material of the socket Pin is the stainless steel 304, and the material of the pull rod 510 is ABS.

As shown in Fig.5, the snare head that the emission electrode 2 formed around the target object can be elliptical, hexagonal or half-moon-shaped so as to adapt to different target object shapes for cutting. Further, the head of the snare can have a tip that easy to shrink, and the guide wires on two sides of the tip are fitted to each other to guide the snare when it is shrunk into the sheath, thereby facilitating the shrink and opening of the snare.

The length of the loop electrode 502 can be any reasonable value, such as 4 to 5 mm. The distance between one end of the loop electrode 502 close to the top of the tube sheath 503 and the top end face of the tube sheath 503 can be any reasonable value, for example, 2 to 3 mm. A water outlet 511 (or referred to as an infusion port) is formed in the top end face of the tube sheath 503.

The emission electrode 501 is telescopically arranged through the slide block 508. In an initial state, the emission electrode 501 is retracted into the tube sheath 503 to facilitate the entry of the front end of the tube sheath 503 into the human body. After arriving at a designated position, the slide block 508 moves forward to push the emission electrode 501 out of the top end face of the tube sheath 503. The slide block 508 is at the socket hole, and the socket Pin 509 is arranged in the socket hole. Fig.6-Fig.8 are partially enlarged or cross section schematic diagrams of a low temperature polypectomy snare surgical device according to a preferred embodiment of the invention. Fig.6 shows a partially enlarged schematic diagram of a bipolar electrode socket connector (snare connector) 600, including: an emission electrode conducting wire (snare head conducting wire) 601 and a loop electrode conducting wire 602. The low temperature polypectomy snare surgical device can be applied to the snare cutting, ablation, coagulation and hemostasis of such as polyps or prominent tumor sites in the digestive tract. In addition, the low temperature polypectomy snare surgical device further can be applied to the snare cutting, ablation, coagulation and hemostasis of soft tissues in joint, spine, skin, ear-nose-throat and other surgeries. The low temperature polypectomy snare surgical device of the present application is used within 24 hours, belongs to temporary contact if being classified according to contact time, belongs to an external access instrument (with tissues/bones/dentine) if being classified according to the nature of the contacted human body, and belongs to active medical equipment if being classified according to the structural characteristics of the medical equipment. An accessory, namely, a bipolar surgical electrode of the low temperature polypectomy snare surgical device is a disposable sterile product.

The low temperature polypectomy snare surgical device adopts a bipolar solution and works at a frequency of 110 kHz. The plasma therapeutic apparatus 100 realizes the snare cutting, ablation, coagulation and hemostasis of soft tissues in the surgeries such as the ear-nose-throat surgery. During the work, the low temperature polypectomy snare surgical device employs normal saline as conductive liquid and forms a thin layer while activating an emission electrode and a loop electrode. When the plasma therapeutic apparatus gives sufficient energy (voltage), the normal saline is converted into a gas layer (plasma layer) composed of energized charged particles. That is, the low temperature polypectomy snare surgical device utilizes energy to excite a conductive medium (e.g., normal saline) to generate plasma, and relies on the energy of the plasma to break tissue molecular bonds. The energy of the plasma directly cleaves biomacromolecules such as proteins into gases such as O², CO², N² and the like, thereby completing vaporization snare cutting of the tissue. When a low voltage is applied to a working knife head of the low temperature polypectomy snare surgical device, an electric field is lower than the threshold requirement for generating the plasma layer, and resistance heat of the tissue is generated, thereby achieving ablation, coagulation and hemostasis of the tissue.

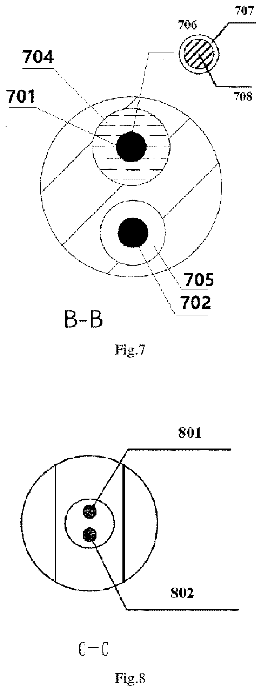

Fig.7 shows a cross section schematic diagram along B-B, including an emission electrode conducting wire (snare head conducting wire) 701, a loop electrode conducting wire 702, a liquid injection cavity 704 and a loop electrode cavity 705. In more detail, Fig.7 shows a section view 706 of the emission electrode conducting wire (snare head conducting wire) 701, including an insulating layer 707 and a metal wire 708. The emission electrode conducting wire (snare head conducting wire) 701 requires the insulating layer for insulating and thermal insulation, and the loop electrode conducting wire 702 may not be provided with the insulating layer.

In addition, those skilled in the art can also use the loop electrode cavity 705 as the liquid injection cavity as needed. When the loop electrode cavity 705 is used as the liquid injection cavity, the loop electrode conducting wire 702 needs to be provided with the insulating layer.

Fig.8 shows a cross section schematic diagram along B-B, including an emission electrode conducting wire (snare head conducting wire) 801 and a loop electrode conducting wire 802.

The present invention has been described with reference to a few embodiments. However, it is well known to those skilled in the art that, as defined by the appended claims of the patent, other embodiments of the present invention in addition to those disclosed above are equally within the scope of the present invention.

In general, all terms used in the claims are interpreted according to their ordinary meanings in the technical field, unless otherwise explicitly defined. All references to "a/the/the [device, component and the like]" are openly interpreted as at least one instance of the device, the component and the like, unless explicitly stated otherwise. The steps of any method disclosed herein are not necessarily required to be performed in the precise order disclosed, unless explicitly stated.

## Claims

1. A low temperature polypectomy snare surgical device, comprising:
a liquid input unit, configured to input liquid to a target object in response to a liquid input signal to form a thin layer of a conductive medium between an emission electrode and a loop electrode;
a bipolar electrode socket connector, connected with a high frequency generator through a high frequency cable to receive a first input voltage generated by the high frequency generator;
the emission electrode, configured to receive the first input voltage generated by the high frequency generator via the bipolar electrode socket connector and apply a first voltage between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma; and
the loop electrode, introduced through the same catheter as the emission electrode and forming a conductive loop in the target object.

2. The low temperature polypectomy snare surgical device according to claim 1, wherein the bipolar electrode socket connector receives a second input voltage generated by the high frequency generator and transmits the second input voltage to the emission electrode, and applies a second voltage between the emission electrode and the loop electrode, so that the target object is maintained at a second temperature to cause ablation and coagulation of the target object.

3. The low temperature polypectomy snare surgical device according to claim 1, wherein the emission electrode is connected to the bipolar electrode socket connector through an emission electrode conducting wire, the emission electrode conducting wire is coated with an insulating layer, and the insulating layer is configured for insulating and thermal insulation.

4. The low temperature polypectomy snare surgical device according to claim 1, further comprising a liquid injection cavity for inputting the liquid to the liquid input unit based on a liquid input instruction, wherein the liquid input unit measures a current residual amount of the liquid in real time and sends the current residual amount to a control unit, and the control unit determines whether to generate the liquid input instruction based on the current residual amount and sends the liquid input instruction to the liquid input unit after determining to generate the liquid input instruction.

5. The low temperature polypectomy snare surgical device according to claim 4, wherein the liquid input unit performs liquid input in one of a titration mode and a continuous liquid supply mode, and the liquid injection cavity is an annular cavity located at the outside of the loop electrode.

6. The low temperature polypectomy snare surgical device according to claim 1, wherein the snare head of the emission electrode is elliptical, hexagonal or half-moon-shaped.

7. The low temperature polypectomy snare surgical device according to claim 6, wherein the liquid input port of the liquid input unit is located between the emission electrode and the loop electrode.

8. The low temperature polypectomy snare surgical device according to claim 1, further comprising a pull rod for enabling an operator to provide a supporting force by holding the pull rod, wherein the low temperature polypectomy snare surgical device further comprises an outer tube for providing an outer layer coating function.

9. The low temperature polypectomy snare surgical device according to claim 1, wherein the emission electrode is telescopically arranged through a slide block.

10. The low temperature polypectomy snare surgical device according to claim 1, wherein the range of the first voltage is 100 Vrms to 300 Vrms, and the range of the second voltage is 60 Vrms to 80 Vrms.

11. A low temperature polypectomy snare surgical system, comprising:
an input unit, configured to receive a control instruction input by a user and send the control instruction to a control unit;
the control unit, configured to parse the control instruction, generate a first mode instruction when the control instruction indicates a first mode, calculate the output power in the first mode according to the current impedance and the control instruction, and send the first mode instruction and a first voltage indication associated with the output power in the first mode to an interface unit;
the interface unit, configured to receive the first mode instruction and the first voltage indication from the control unit, forward the first mode instruction and the first voltage indication to a plasma unit, receive the current impedance of a target contact end from a plasma unit and send the current impedance to the control unit; and
the plasma unit, configured to enter the first mode in response to the reception of the first mode instruction and the first voltage indication from the interface unit, in which mode circuit activation is performed between an emission electrode and a loop electrode at the target contact end of the plasma unit via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma;
wherein the emission electrode, the plasma layer, the loop electrode and the target contact end form a loop.

12. The low temperature polypectomy snare surgical system according to claim 11, wherein the control unit further parses the control instruction, generates a second mode instruction when the control instruction indicates a second mode, calculates the output power in the second mode according to the current impedance and the control instruction, and sends the second mode instruction and the second voltage indication associated with the output power in the second mode to the interface unit.

13. The low temperature polypectomy snare surgical system according to claim 12, wherein the interface unit receives the second mode instruction and the second voltage indication from the control unit and forwards the second mode instruction and the second voltage indication to the plasma unit.

14. The low temperature polypectomy snare surgical system according to claim 13, wherein the plasma unit enters the second mode in response to the reception of the second mode instruction and the second voltage indication from the interface unit, applies a second voltage to maintain the target contact end of the plasma unit at a second temperature so as to perform ablation and coagulation of the target object.

15. The low temperature polypectomy snare surgical system according to claim 11, further comprising an alarm unit, configured to perform alarm via voice prompt, character prompt and/or indicator lamp display when an alarm signal is received;
wherein the plasma unit sends an alarm signal to the control unit after detecting an operation fault, and the control unit sends the alarm signal to the alarm unit.

16. The low temperature polypectomy snare surgical system according to claim 11, wherein the input unit is a pedal type input device, wherein the user generates the control instruction by operating the pedal type input device, and the control instruction is a two-tuples <mode, power>.

17. The low temperature polypectomy snare surgical system according to claim 11, further comprising a dropping liquid input unit for inputting the conductive medium to the plasma unit based on a conductive medium input instruction of the control unit, wherein the plasma unit measures a current residual amount of the conductive medium in real time and sends the current residual amount to the control unit, and the control unit determines whether to generate the conductive medium input instruction based on the current residual amount and sends the conductive medium input instruction to the dropping liquid input unit after determining to generate the conductive medium input instruction.

18. The low temperature polypectomy snare surgical system according to claim 11, further comprising a display unit for displaying an operation state of the low temperature polypectomy snare surgical system in real time.

19. The low temperature polypectomy snare surgical system according to claim 11, wherein the range of the first voltage is 100 Vrms to 300 Vrms, and the range of the second voltage is 60 Vrms to 80 Vrms.

20. The low temperature polypectomy snare surgical system according to claim 11, wherein the range of the first temperature is 35°C-40°C, and the range of the second temperature is 40°C-70°C, and
in the first mode, a thermal penetration distance is less than or equal to 150 microns, and in the second mode, the thermal penetration distance is less than or equal to 200 microns.

21. A low temperature polypectomy snare surgical method, comprising:
receiving a control instruction input by a user;
parsing the control instruction, generating a first mode instruction when the control instruction indicates a first mode, calculating the output power in the first mode according to the current impedance and the control instruction, and determining a first voltage indication associated with the output power in the first mode;
forwarding the first mode instruction and the first voltage indication to a plasma device, and
receiving the current impedance of a target contact end from the plasma device;
causing the plasma device to enter the first mode in response to the reception of the first mode instruction and the first voltage indication, in which mode circuit activation is performed between an emission electrode and a loop electrode at the target contact end of the plasma unit via a conductive medium to form a thin layer, and a first voltage is applied between the emission electrode and the loop electrode, such that the conductive medium reaches a first temperature and is converted into a plasma layer, thereby exciting the conductive medium with electrical energy to generate plasma and performing vaporization snare cutting on the target object based on the radio frequency energy of the plasma;
wherein the emission electrode, the plasma layer, the loop electrode and the target contact end form a loop.

22. The method according to claim 21, further comprising: parsing the control instruction, generating a second mode instruction when the control instruction indicates a second mode, calculating the output power in the second mode according to the current impedance and the control instruction, and determining a second voltage indication associated with the output power in the second mode.

23. The method according to claim 22, further comprising: forwarding the second mode instruction and the second voltage indication to the plasma device.

24. The method according to claim 23, further comprising: causing the plasma device to enter the second mode in response to the reception of the second mode instruction and the second voltage indication, in which mode a second voltage is applied to maintain the target contact end of the plasma unit at a second temperature so as to perform ablation and coagulation of the target object.

25. The method according to claim 21, further comprising: performing alarm via voice prompt, character prompt and/or indicator lamp display when an alarm signal is received; and
generating the alarm signal after detecting an operation fault.

26. The low temperature polypectomy snare surgical method according to claim 21, wherein the user generates the control instruction by operating a pedal type input device, and the control instruction is a two-tuples <mode, power>.

27. The low temperature polypectomy snare surgical method according to claim 21, further comprising: inputting the conductive medium to the plasma device based on a conductive medium input instruction, wherein the plasma unit measures a current residual amount of the conductive medium in real time and determines whether to generate the conductive medium input instruction based on the current residual amount.

28. The low temperature polypectomy snare surgical method according to claim 21, further comprising: displaying an operation state of the plasma device in real time.

29. The low temperature polypectomy snare surgical method according to claim 21, wherein the range of the first voltage is 100 Vrms to 300 Vrms, and the range of the second voltage is 60 Vrms to 80 Vrms.

30. The low temperature polypectomy snare surgical method according to claim 21, wherein the range of the first temperature is 35°C-40°C, and the range of the second temperature is 40°C-70°C, and
in the first mode, a thermal penetration distance is less than or equal to 150 microns, and in the second mode, the thermal penetration distance is less than or equal to 200 microns.
